# EUROPEAN PATENT APPLICATION

(11) **EP 4 489 012 A1**
(43) Date of publication of application: **08.01.2025**
(21) Application number: 23770238.6
(22) Date of filing: 16.02.2023
(51) Int. Cl.: G16C 20/30

(54) **INFORMATION PROCESSING METHOD AND INFORMATION PROCESSING SYSTEM**

(30) Priority: 14.03.2022 JP 2022038898
(71) Applicant: Sony Group Corporation, Tokyo 108-0075 (JP)
(72) Inventor: TAKI, Yuko, Tokyo 108-0075 (JP); INAGAKI, Yasuhito, Atsugi-shi, Kanagawa 243-0014 (JP); YAMAGUCHI, Yoko, Tokyo 108-0075 (JP); FUJINAGA, Takashi, Tokyo 108-0075 (JP)
(74) Representative: D Young & Co LLP
(86) International application number: PCT/JP2023/005360
(87) International publication number: WO 2023/176278

(57) **Abstract**

A main object of the present disclosure is to provide an information processing technology capable of outputting useful information for effectively utilizing a product as a new resource.

The present disclosure provides an information processing method executed by one or more processors, the method including: specifying at least one of information regarding a characteristic of a product described in the following (a) to (c) on the basis of a name of a chemical substance and/or a functional group contained in the product; and outputting the information regarding the characteristic of the product specified.
(a) Information regarding characteristics different from the original characteristics of the product.
(b) Information regarding characteristics that the product can acquire as it changes over time.
(c) Information regarding characteristics obtainable by modifying the product.

## Description

### TECHNICAL FIELD

The present disclosure relates to an information processing method and an information processing system. Specifically, the present invention relates to an information processing method and an information processing system capable of outputting specific information regarding characteristics of a product.

### BACKGROUND ART

Conventionally, various techniques related to waste recycling have been proposed.

For example, Patent Document 1 below discloses a technique for improving the accuracy of waste classification for the purpose of improving the material quality after horizontal recycling. Chemical recycling requires cost for reducing to a monomer, and material recycling requires cost for reducing to a polymer. These costs are more expensive than when the same good is constituted by a virgin material. The cost of recycling is generally borne by the consumer as a social cost or by the emitter by handing the waste to the recycler for an inverse fee (a transaction in which the flow of money and material is in the same direction). Furthermore, the material after horizontal recycling is at best of similar quality to virgin material, and for the most part is of lower quality. Due to the mechanism, the horizontally recycled material is a material having a higher cost and a quality lower than that of the virgin material.

Patent Document 2 below discloses a search system for facilitating recycling. In the search system, when a user inputs a type or a component of an unused substance as a keyword, a recycling technology including a keyword and a similar word of the keyword is extracted from a database. However, the manufacturer can know the details of the contained components or compositions of the unused substances, but the user often cannot know the details. For example, in the case of a plastic molded body, the user can infer the polymer of the main component from the use and the like, but often does not know the details. This is because it is necessary to perform a plurality of chemical analyses in order to grasp the type and amount of the additive contained in the plastic molded body, but it is often difficult for a user to perform the chemical analysis.

### CITATION LIST

### PATENT DOCUMENT

Patent Document 1: Japanese Patent Application Laid-Open No. H7-24437
Patent Document 2: Japanese Patent Application Laid-Open No. 2004-310661

### SUMMARY OF THE INVENTION

### PROBLEMS TO BE SOLVED BY THE INVENTION

Waste is steadily increasing. In order to reduce the amount of waste, new technologies for effectively utilizing products as new resources are required.

Therefore, a main object of the present disclosure is to provide an information processing technology capable of outputting useful information for effectively utilizing a product as a new resource.

### SOLUTIONS TO PROBLEMS

That is, the present disclosure provides
an information processing method executed by one or more processors, the method including:
specifying at least one of information regarding a characteristic of a product described in following (a) to (c) on the basis of a name of a chemical substance and/or functional group contained in the product:
   (a) information regarding a characteristic different from an original characteristic of the product;
   (b) information regarding a characteristic that the product can acquire as it changes over time; and
   (c) information regarding a characteristic obtainable by modifying the product;
      and
outputting the information regarding the characteristic of the product specified.

The information processing method may further include receiving information regarding a product.

The information regarding the product may include attribute information of the product and/or image information using the product as a subject.

The information processing method may further include acquiring a name of a chemical substance and/or a functional group contained in the product received with reference to a database holding information regarding the product and the name of the chemical substance and/or the functional group contained in the product in association with each other.

The information processing method may further include registering the information regarding the product received and the name of chemical substance and/or the functional group contained in the product received in the database in association with each other.

In the information processing method, the information regarding the characteristic of the product may include information regarding a function and/or use of the product.

The outputting the information regarding the characteristic of the product specified may include outputting information regarding a good using a function of the product and/or information regarding a good according to a use of the product.

The outputting the information regarding the characteristic of the product specified may include extracting information of an output target on the basis of a characteristic of a good from among information regarding a good using a function of the product and/or information regarding a good according to a use of the product, creating a list including the information extracted of the output target, and outputting the list.

The information processing method may further include receiving attribute information of a user.

The outputting the information regarding the characteristic of the product specified may include extracting information of an output target on the basis of a characteristic of a good and attribute information of the user from among information regarding a good using a function of the product and/or information regarding a good according to a use of the product, creating a list including the information extracted of the output target, and outputting the list.

The information processing method may further include:
receiving one or more pieces of information selected by the user from among the information regarding the characteristic of the product specified;
performing machine learning using teacher data in which attribute information of the user and the one or more pieces of information selected by the user are associated with each other; and
generating, in a case where attribute information of a user is input, a learned model for extracting information of an output target from among the information regarding the characteristic of the product specified.

The outputting the information regarding the characteristic of the product specified may include outputting information obtained by inputting attribute information of the user to the learned model.

The product may be an unnecessary product.

The product may be a waste.

Further, the present disclosure provides
an information processing system including:
one or more processors; and one or more memories storing instructions that, when executed by the one or more processors, cause the information processing system to perform operations,
in which the operations comprise:
   specifying at least one of information regarding a characteristic of a product described in following (a) to (c) on the basis of a name of a chemical substance and/or functional group contained in the product:
      (a) information regarding a characteristic different from an original characteristic of the product;
      (b) information regarding a characteristic that the product can acquire as it changes over time; and
      (c) information regarding a characteristic obtainable by modifying the product;
         and
   outputting the information regarding the characteristic of the product specified.

### BRIEF DESCRIPTION OF DRAWINGS

Fig. 1 is a diagram illustrating an example of a system configuration of an information processing system according to a first embodiment.
Fig. 2 is a diagram illustrating an example of a hardware configuration of a server.
Fig. 3 is a diagram illustrating an example of a hardware configuration of a user terminal.
Fig. 4 is a flowchart illustrating an example of an operation of the information processing system according to the first embodiment.
Fig. 5 is a flowchart illustrating an example of processing of registering data in a product information database.
Fig. 6 is a flowchart illustrating an example of processing of registering data in a product information database.
Fig. 7 is a flowchart illustrating an example of processing of registering data in a chemical substance/functional group database.
Fig. 8 is a flowchart illustrating an example of an operation of an information processing system according to a second embodiment.
Fig. 9 is a flowchart illustrating an example of an operation of an information processing system according to a modification.

### MODE FOR CARRYING OUT THE INVENTION

Hereinafter, preferred modes for carrying out the present disclosure will be described. Embodiments described below illustrate representative embodiments of the present disclosure, and the scope of the present disclosure is not limited only to these embodiments.

The present disclosure will be described in the following order.
1. Outline of Present Disclosure
2. Embodiments of Present Disclosure
2-1. First Embodiment
2-1-1. System Configuration
2-1-2. Hardware Configuration
2-1-3. Operation
2-2. Second Embodiment
2-3. Modifications

### 1. Outline of Present Disclosure

As a result of studying a technique for effectively utilizing a product as a new resource, the present inventors have focused on the following points. The product may be chemically altered or other materials may adhere in the course of disposal. Therefore, in the product at the time of disposal, there may be chemical substances and/or functional groups that were not present in the material before processing and at the time of product shipment. Some of such materials containing chemical substances and/or functional groups are traded as functional materials different from the original use of the product.

The present inventors have considered that the waste can be effectively utilized as a functional material by utilizing a function caused by a chemical substance and/or a functional group newly generated in the waste. In addition, the present inventors have considered that there is a case where the modified waste can be effectively utilized as a functional material by using a chemical substance and/or a functional group contained in the waste as a starting point of the modification. On the basis of such an idea, the present inventors have considered that useful information for effectively utilizing a product as a new resource is included in information obtained on the basis of a chemical substance and/or a functional group contained in the product, and have further studied. As a result, the present inventors have found that at least one of the following information (a) to (c) specified on the basis of the names of chemical substances and/or functional groups contained in a product is useful for effectively utilizing the product as a new resource.
(a) Information regarding characteristics different from the original characteristics of the above-described product.
(b) Information regarding characteristics that the above-described product can acquire as it changes over time.
(c) Information regarding characteristics obtainable by modifying the above-described product.

That is, the present disclosure provides an information processing technology that outputs at least one of the pieces of information regarding the characteristics of the above-described product of (a) to (c) described above on the basis of the name of the chemical substance and/or the functional group contained in the product.

In the present disclosure, a "product" refers to an object made by modifying raw materials. The product includes not only a finished product created through a series of manufacturing processes but also a semifinished good, a part, an accessory, a defective, a defective product, a failed product, a prototype, an end material, a surplus material, and a raw material created by combining a plurality of raw materials generated in a middle stage of the manufacturing process or in a development process. The product may be, for example, an unused product or a product that has been used once or more. The product may be, for example, a commercial product or may not be a commercial product. The product may be, for example, an inventory, a sample, an exhibit, or the like.

The product in the present disclosure may be, for example, an unnecessary product. In the present disclosure, "unnecessary product" refers to a product that is judged to be unnecessary by a person who has made the product or a person who has owned the product, a product that does not serve its original purpose, or a product that is generally judged to be difficult to serve its original purpose.

The product in the present disclosure may be, for example, waste. In the present disclosure, "waste" refers to a product discarded by a person who has made the product or a person who has owned the product, or a product generally determined to be discarded. The waste may be, for example, sorted waste or unsorted waste.

According to the present disclosure, it is possible to obtain useful information for effectively utilizing a product including an unnecessary product and waste as a new resource.

### 2. Embodiments of Present Disclosure

### 2-1. First Embodiment

An information processing system according to a first embodiment of the present disclosure will be described.

### 2-1-1. System Configuration

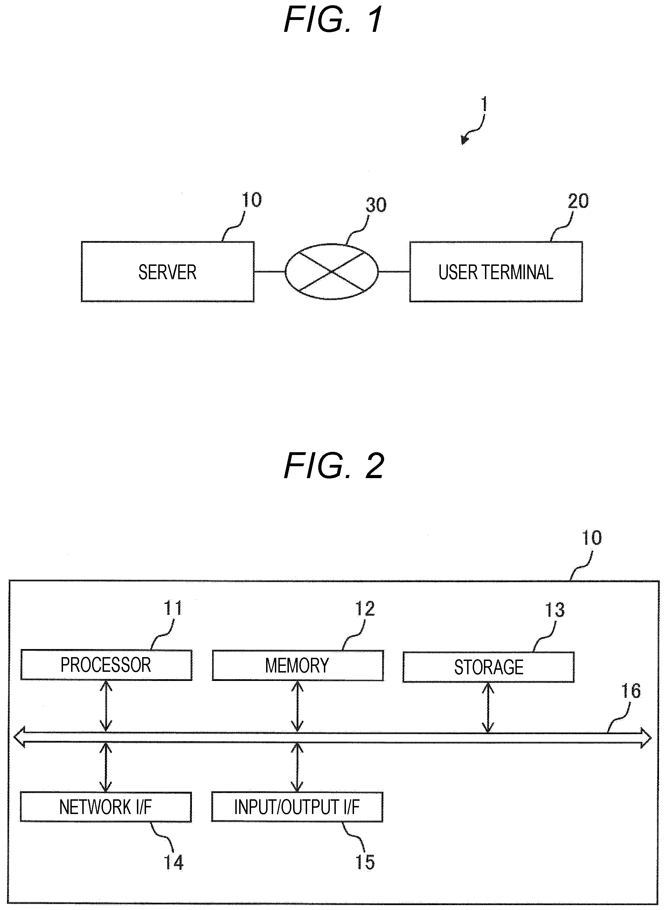

The information processing system according to the present embodiment includes one or more information processing apparatuses. An example of a system configuration of an information processing system 1 according to the present embodiment will be described with reference to Fig. 1. The information processing system 1 includes a server 10 and a user terminal 20. The server 10 and the user terminal 20 are communicably connected via a network 30. In the present disclosure, the server 10 is an example of an information processing apparatus, and may be expressed as, for example, a first information processing apparatus. The user terminal 20 is another example of the information processing apparatus, and may be expressed as, for example, a second information processing apparatus.

The server 10 executes processing including specifying information regarding a characteristic of a product. Although one server 10 is illustrated in the figure, the number of servers 10 is not limited thereto. The server 10 may be one or a plurality of physical servers, or may be one or a plurality of virtual servers built on one or a plurality of physical servers. The plurality of physical servers described above may be disposed at the same place geographically, or may be disposed geographically dispersedly.

The user terminal 20 may be used, for example, for the user to input and refer to information regarding a product. The user terminal 20 may be, for example, a desktop type, a laptop type, or a tablet type personal computer, a smartphone, a mobile phone (feature phone), or the like. Although one user terminal 20 is illustrated in Fig. 1, the number of user terminals 20 is not limited thereto.

The network 30 is a communication network through which data is transmitted and received, and may be, for example, the Internet, a telephone network, a mobile communication network, a sanitary communication network, a dedicated line network, a local area network (LAN), or a combination thereof. The network 30 may be a wired communication network, a wireless communication network including Wi-Fi (registered trademark) and Bluetooth (registered trademark), or a combination thereof.

### 2-1-2. Hardware Configuration

An example of a hardware configuration of the server 10 will be described with reference to Fig. 2. The server 10 comprises one or more processors 11 and one or more memories 12.

The processor 11 controls the operation of the server 10. Examples of the processor 11 include a central processing unit (CPU).

The memory 12 stores instructions that, when executed by the processor 11, cause the server 10 to perform operations. Examples of the memory 12 include computer memories such as a read only memory (ROM) and a random access memory (RAM).

The server 10 may further include a storage 13, a network interface (network I/F) 14, and an input/output interface (input/output I/F) 15.

The storage 13 stores information and programs related to the operation and use of the server 10. Examples of the storage 13 include a hard disk drive (HDD) and a solid state drive (SSD).

The network I/F14 is an interface for connecting to the network 30. The network I/F14 enables the server 10 to transmit and receive various data via the network 30.

The input/output I/F15 is an interface for connecting to an input/output apparatus. An input apparatus and an output apparatus are connected to the input/output I/F15. The input apparatus may be any one of all apparatuses capable of accepting input of various data to the server 10 or a combination thereof. Examples of the input apparatus include a keyboard, a mouse, a touch panel, and a button interface. The output apparatus may be any one of all apparatuses capable of outputting various data or a combination thereof. Examples of the output apparatus include a display, a printer, and a speaker.

The processor 11, the memory 12, the storage 13, the network I/F14, and the input/output I/F15 are mutually connected by, for example, a bus 16. In Fig. 2, although any hardware is illustrated as a single piece, it is merely an example, and any hardware may be one or more. Furthermore, in Fig. 2, all the hardware is provided in the single server 10, but this is merely an example, and the hardware may be provided in a distributed manner in a plurality of apparatuses.

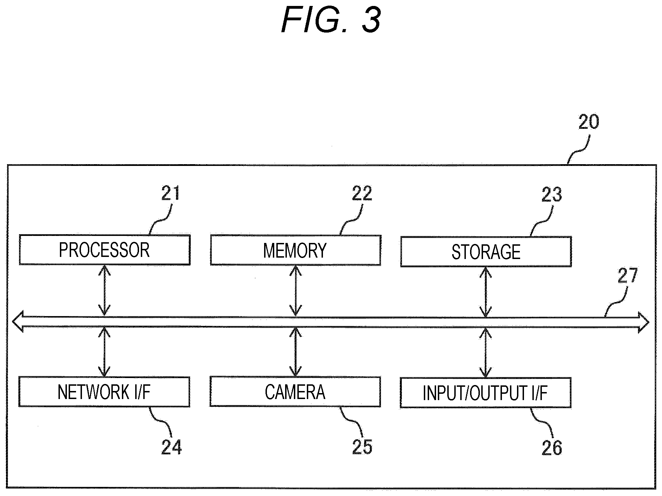

An example of a hardware configuration of the user terminal 20 will be described with reference to Fig. 3. The user terminal 20 may include a processor 21, a memory 22, a storage 23, a network interface (network I/F) 24, a camera 25, and an input/output interface (input/output I/F) 26.

The processor 21 controls an operation of the user terminal 20. Examples of the processor 21 include a central processing unit (CPU).

The memory 22 stores instructions that, when executed by the processor 21, cause the user terminal 20 to perform operations. Examples of the memory 22 include computer memories such as a read only memory (ROM) and a random access memory (RAM).

The storage 23 stores information and programs related to the operation and use of the user terminal 20. Examples of the storage 23 include a hard disk drive (HDD) and a solid state drive (SSD).

The network I/F24 is an interface for connecting to the network 30. The network I/F24 enables the user terminal 20 to transmit and receive various data via the network 30.

The camera 25 includes, for example, an imaging element such as a charge coupled device (CCD) or a complementary metal oxide semiconductor (CMOS). The camera 25 may be used, for example, to acquire image information with a product as a subject.

The input/output I/F26 is an interface for connecting to an input/output apparatus. An input apparatus and an output apparatus are connected to the input/output I/F26. The input apparatus may be any one of all apparatuses capable of accepting input of information to the user terminal 20 or a combination thereof. Examples of the input apparatus include a keyboard, a mouse, a touch panel, and a button interface. The output apparatus may be any one of all apparatuses capable of outputting various data or a combination thereof. Examples of the output apparatus include a display, a printer, and a speaker.

The processor 21, the memory 22, the storage 23, the network I/F24, the camera 25, and the input/output I/F26 are mutually connected by, for example, a bus 27. In Fig. 3, although any hardware is illustrated as a single piece, it is merely an example, and any hardware may be one or more. Furthermore, in Fig. 3, all the hardware is provided in the single user terminal 20, but this is merely an example, and the hardware may be distributed and provided in a plurality of apparatuses.

In the present embodiment, one or more processors included in the information processing system 1 (specifically, one or more information processing apparatuses such as the server 10 and the user terminal 20) execute the information processing program according to the present embodiment and execute the operation described below, thereby implementing an information processing method including the operation. That is, the information processing method according to the present embodiment is an information processing method executed by one or more processors. Furthermore, the information processing system 1 (specifically, one or more information processing apparatuses such as the server 10 and the user terminal 20) according to the present embodiment includes one or more processors and one or more memories that store commands that, when executed by the one or more processors, cause the information processing system 1 to execute operations described below, and executes the information processing program according to the present embodiment using these.

The information processing program according to the present embodiment may be stored in various types of non-transitory computer readable media and supplied to the information processing apparatus. The non-transitory computer readable media include various types of tangible storage media. Examples of the non-transitory computer readable medium include a magnetic recording medium (for example, a flexible disk, a magnetic tape, and a hard disk drive), a magneto-optical recording medium (for example, a magneto-optical disk), a CD-ROM, a CD-R, a CD-R/W, and a semiconductor memory (for example, a mask ROM, a programmable ROM (PROM), an erasable PROM (EPROM), a flash ROM, and a RAM). Furthermore, the above-described information processing program may be supplied to the information processing apparatus by various types of transitory computer readable media. Examples of transitory computer readable media include electrical signals, optical signals, and electromagnetic waves. The transitory computer readable medium can supply the above-described information processing program to the information processing apparatus via a wired communication path such as an electric wire and an optical fiber, or a wireless communication path.

### 2-1-3. Operation

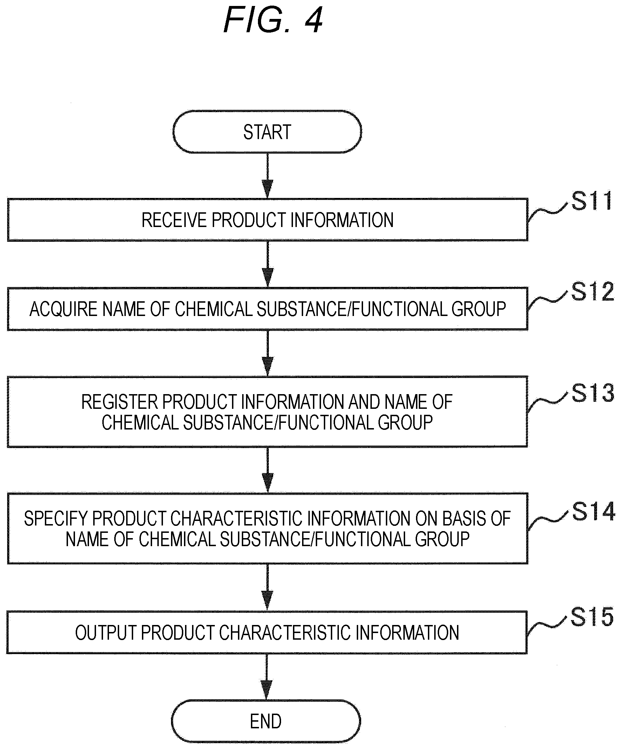

An example of the operation of the information processing system 1 will be described with reference to Fig. 4. First, the server 10 receives information regarding a product (hereinafter, also referred to as "product information") (step S11). The processing in step S11 may be, for example, that the server 10 receives the product information input by the user to the user terminal 20 from the user terminal 20, or that the server 10 directly receives the input of the product information from the user.

The product information is information used to specify a product. The server 10 can specify the product on the basis of the received product information. The product information includes, for example, attribute information of the product and/or image information of the product as a subject. The attribute information of the product may include, for example, a name, a serial number, a part number, characteristics (for example, functions and uses), a shape, a size, a weight, a main component, a manufacturer, a manufacturing place, a manufacturing date, a manufacturing quantity, a transaction price of the product in a market, and the like. In a case where the product is a waste, the attribute information may further include a person who disposed of it, a disposal place, a disposal date, a disposal amount, a person who collected it, a place of collection, a date of collection, a transaction price of the waste in a market, information regarding an environmental load caused by the waste, information regarding a grant for waste treatment, regulatory information regarding the waste in each country, and the like. The image information with the product as the subject may be acquired using, for example, a camera included in the user terminal 20, but is not limited thereto. The image information with the product as a subject may be acquired by an arbitrary imaging apparatus. In a case where the product information includes only image information in which a product is a subject, the server 10 can specify the product of the subject using, for example, an image recognition technology.

Next, the server 10 acquires names of chemical substances and/or functional groups (hereinafter, also referred to as "chemical substance/functional group") contained in the above-described product (step S12). As an example, the processing in step S12 may be that the server 10 receives, from the user terminal 20, the name of the chemical substance/functional group included in the product input by the user to the user terminal 20. In this case, the name of the chemical substance/functional group contained in the product may be obtained from the chemical analysis result of the product, or may be obtained by the user from a document or the like. As another example, the processing in step S12 may be that the server 10 acquires the names of chemical substances and/or functional groups contained in the product from information on the Internet. As another example, the processing in step S12 may be that the server 10 refers to a database (hereinafter, also referred to as a "product information database") that holds the above-described product information and names of chemical substances/functional groups included in the product in association with each other, and acquires the names of chemical substances/functional groups included in the product.

Next, the server 10 registers the product information and the name of the chemical substance/functional group included in the product in the product information database in association with each other as necessary (step S13). For example, in a case where the product information and the name of the chemical substance/functional group are not registered in the product information database, the process of step S13 may be executed. As a result, the information held in the product information database is updated.

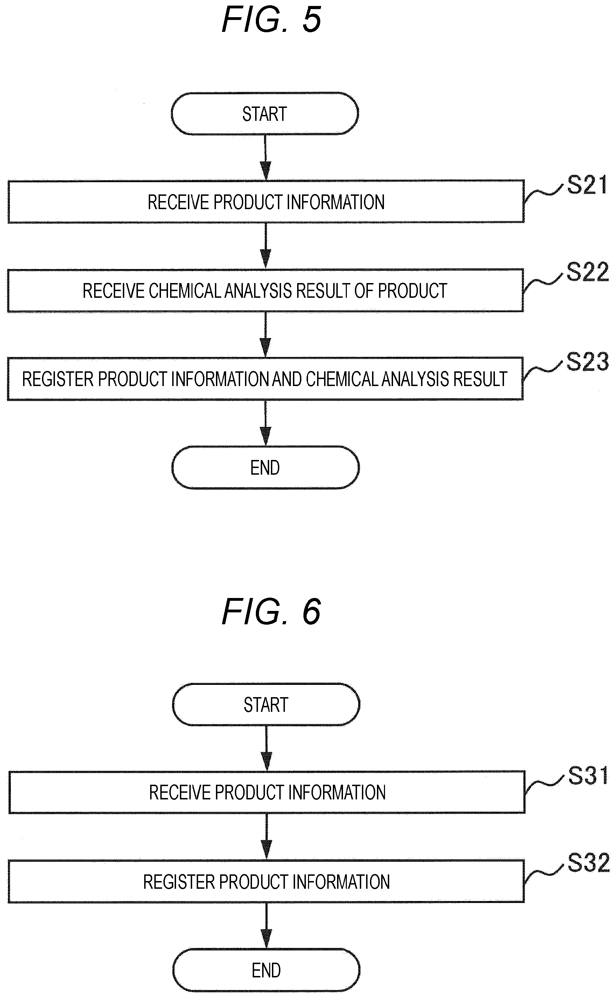

Here, an example of a process of registering data in the product information database will be described with reference to Figs. 5 and 6. As illustrated in Fig. 5, the server 10 receives product information (step S21), and then receives a chemical analysis result of the product (step S22). The chemical analysis result includes the name of the chemical substance/functional group contained in the product. The server 10 registers the product information received in step S21 and the chemical analysis result (including the name of the chemical substance/functional group contained in the product) received in step S22 in the product information database in association with each other (step S23). Through these processes, the product information database can be constructed and updated.

In addition, as illustrated in Fig. 6, the server 10 may receive product information (step S31), and then register the product information in the product information database (step S32). That is, only product information not associated with a chemical analysis result may be registered in the product information database. In this case, when the chemical analysis result is later registered in association, the processing of step S21 illustrated in Fig. 5 may not be performed, and the processing of steps S22 and S23 is only required to be performed.

As another example of the processing of registering data in the product information database, the server 10 may acquire the name of the chemical substance/functional group contained in the product from information on the Internet and register the name of the chemical substance/functional group in the product information database. In addition, as another example, in step S22, the server 10 may receive the name of the chemical substance/functional group obtained by the user from a document or the like instead of the chemical analysis result of the product.

The product information database may be stored in the server 10 (for example, the storage 13) or may exist outside the server 10.

An example of the operation of the information processing system 1 will be continuously described with reference to Fig. 4 again. On the basis of the name of the chemical substance/functional group contained in the product acquired in step S12, the server 10 specifies at least one of the following (a) to (c) information regarding the characteristics of the product (hereinafter, also referred to as "product characteristic information") (step S14).
(a) Information regarding characteristics different from the original characteristics of the product.
(b) Information regarding characteristics that the product can acquire as it changes over time.
(c) Information regarding characteristics obtainable by modifying the product.

The above-described product characteristic information may include, for example, information regarding the function and/or use (hereinafter, also referred to as "function/use") of the product, information regarding the good using the function of the product, and information regarding the good according to the use of the product. In the present disclosure, a "good" refers to an object created for the purpose of business regardless of whether it is for profit or for free. For example, if the function is a function of repelling an organism, the good utilizing the function may be a repellent. For example, if the use is a foam, a good suitable for the use may be a heat insulating material. The product characteristic information may include character information, or may include other information such as image information or audio information in addition to the character information.

Among the characteristics illustrated in the above (a) to (c), first, characteristics different from the initial characteristics of the product illustrated in the above (a) will be described. The "initial" in the "characteristic different from the original characteristic of the product" refers to a time point at which the product is produced for the original purpose of use or a time point at which the product is produced. For example, there is a case where the original purpose of use (the purpose of use of the non-defective product) has not been achieved at the time of manufacture of the defective product, but in this case, the time point at which the defective product occurs can be the "initial" of the defective product.

The characteristics different from the original characteristics of the product will be described with an example. For example, at a manufacturing site of a plastic molded article, an operation of mixing a resin raw material and various additives to prepare a molding plastic and then molding the molding plastic can be performed. End materials (sprue, runner, burr, and the like) generated in molding the plastic are removed after molding. The removed end material is heated and melted after pulverization, and can be used again as a raw material of the molded article. However, the repeatedly utilized, i.e., repeatedly heated, plastic gradually deteriorates. For example, the flowability of the plastic is changed by oxidative decomposition to change the molecular weight, or the plastic is colored by generation of a conjugated double bond. The deteriorated plastic finally cannot be used as a raw material of the molded article. It can be said that such a plastic initially has characteristics suitable for molding, but has lost the original characteristics by being heated and deteriorated.

On the other hand, the plastic may acquire characteristics different from those before heating by heating. For example, in a case where the plastic is oxidatively decomposed by heating, a chemical substance/functional group having a function of repelling or attracting an organism may be generated. It is assumed that the oxidative decomposition as described above occurs by repeatedly heating the molding plastic. The repeatedly heated molding plastic may eventually lose its original characteristic of being suitable for molding. On the other hand, the molding plastic may acquire a characteristic different from the original characteristic of repelling or attracting an organism by a newly generated chemical substance/functional group.

In addition, another example is a headlight cover of an automobile. The headlight cover is generally constituted by polycarbonate. A topcoat agent such as an acrylic resin or a silicone resin is applied to the surface of the polycarbonate. By pulverizing, melting, and kneading the headlight cover, a mixed raw material of polycarbonate as a main component and a topcoat agent can be obtained. The mixed raw material can be a new resin raw material having characteristics different from those of the headlight cover.

In this way, the product may acquire characteristics different from the original characteristics. It is considered that the different characteristics of the product are brought about by chemical substances/functional groups not originally included in the product. Therefore, specifying (a) information regarding characteristics different from the original characteristics of the product on the basis of the name of the chemical substance/functional group contained in the product in above step S14 may include, for example, specifying that the chemical substance/functional group contained in the product was not originally contained.

Next, characteristics that the product can acquire as it changes over time illustrated in the above (b) will be described. "Aging" in the "characteristics that the product can acquire as it changes over time" refers to a change that occurs with the lapse of time. Aging may preferably be a change that occurs with the product being used or left after use. The time for which the product is used or left after use may be, for example, 24 hours or more, 48 hours or more, 72 hours or more, 1 week or more, 1 month or more, half a year or more, 1 year or more, or the like but is not limited thereto. In addition, the time for which the product is used or left after use may be, for example, 10 years or less, 7 years or less, 5 years or less, 4 years or less, 3 years or less, or the like but is not limited thereto.

The "change" in "aging" may be, for example, but not limited to, one or a combination of two or more selected from chemical changes, physical changes, changes in characteristics (for example, color, refractive index, thermal conductivity, and the like), and the like.

Characteristics that the product can acquire as it changes over time will be described by way of example. For example, in a plastic molded article that is a used, non-used, or waste product, oxidative decomposition of the plastic progresses over time, and a chemical substance/functional group having a function of repelling or attracting an organism may be generated. For example, in a plastic molded article that has been used or left for a long period of time outdoors (particularly in the ocean or river), oxidative decomposition of plastic tends to proceed, and a state in which a chemical substance/functional group having a function of repelling or attracting an organism can be generated tends to easily occur. As described above, there is a case where the plastic molded article can acquire a new characteristic of repelling or attracting an organism as it changes over time.

Next, characteristics obtainable by modifying the product illustrated in (c) above will be described with reference to examples. For example, high impact polystyrene (HIPS) is generally used for VHS video cassette cases. Polymeric flocculants can be produced by sulfonating and modifying used VHS video cassette cases (i.e., HIPS). In addition, in the case of an 8-mm video cassette case using an acrylonitrile-butadiene-styrene (ABS) resin, a water-absorbent resin can be produced by simultaneously generating sulfonation and hydrolysis with concentrated sulfuric acid. As described above, the used video cassette case can be modified to be utilized in new uses such as a flocculant or a water-absorbent resin.

The sulfonation of HIPS described above is an example of modification to a molecular structure, specifically, an example of modification of a basic unit of a polymer. Examples of the modification of the basic unit of the polymer include functional group addition reactions such as imidazolination, esterification, hydrolysis, chloromethylation, and hydroxyl group addition, in addition to sulfonation.

Other examples of modifying a polymeric material, such as plastic, are also included. Another example of modification to the molecular structure is modification of the polymer main chain. Examples of the modification of the polymer main chain include an oxidation reaction such as generation of a double bond and extension of conjugation, a high molecular weight due to a crosslinking reaction, and a low molecular weight due to main chain cleavage or hydrolysis by oxidation or thermal decomposition. In addition to the modification to these molecular structures, for example, modification to a molded body raw material such as a pellet and modification to a molded body can also be mentioned. The modification to the molded body raw material such as pellets may be, for example, changing the physical properties of the bulk by heating and melting the polymer material, mixing an additive, or changing crystallinity such as crystallinity and crystallite size in the case of a crystalline polymer. The modification to the molded body may be, for example, changing the physical properties of the surface by a treatment using ozone or ultraviolet rays or a silane treatment using a silane coupling agent.

As exemplified above, the characteristics illustrated in the above (a) to (c) can bring new value as a resource to a product. Therefore, the products can be effectively utilized as new resources by utilizing the characteristics described in the above (a) to (c). The new resources obtained in this manner can have a higher added value than resources obtained by horizontal recycling that has been conventionally performed. Therefore, the new resources are expected to be traded at a higher market price than the resources obtained by horizontal recycling. This motivates the use of products including an unnecessary product and waste as new resources, and it is expected that the reduction of waste and the reduction of environmental load will be promoted in the end.

In conventional horizontal recycling, materials derived from waste are separated and unified, or purified to increase purity. On the other hand, the method for generating a new resource using the characteristics described in the above (a) to (c) does not mainly aim at unifying or improving the purity of a material derived from a product. The method aims to contribute to creation of new resources by taking advantage of features unique to a product that are not present in a single material, for example, features that the product can be a mixture containing various components, can contain chemical changes (for example, deterioration), can contain impurities, and can be non-uniform.

The new resources obtained by utilizing the characteristics described in the above (a) to (c) can be a mixture of various components since the features of the above-described product are utilized. In order to produce such a new resource only from a virgin material, in some cases, a step of synthesizing or purifying the various components is required. It is considered that a new resource utilizing the features of a product can be obtained in less processes than an equivalent conventional resource produced using only a virgin material as a raw material in some cases. As a result, it is considered that resources equivalent to the conventional resources can be provided at a lower cost. In this case, there is an advantage that a purchaser who has purchased the conventional resource can purchase a resource equivalent to the conventional resource at a lower cost.

In the present disclosure, the name of chemical substance/functional group contained in the product is used in order to grasp features unique to the product that are not included in the virgin material. Specifically, as described above, the server 10 specifies the product characteristic information on the basis of the name of the chemical substance/functional group contained in the product (step S14 in Fig. 4).

In order to specify the product characteristic information on the basis of the name of the chemical substance/functional group, the server 10 may refer to, for example, the above-described product information database that holds the product information and the name of the chemical substance/functional group included in the product in association with each other. In this case, the product information database may further hold, for example, a name of a chemical substance/functional group originally contained in the product, a name of a chemical substance/functional group that can be newly generated in the product, a name of a chemical substance/functional group that can be generated as it changes over time of the product, and a name of a chemical substance/functional group that can be generated by modification of the product.

Furthermore, in order to specify the product characteristic information on the basis of the name of the chemical substance/functional group, the server 10 may refer to, for example, a database (hereinafter, also referred to as a "chemical substance/functional group database") that holds the name of the chemical substance/functional group and the information regarding the feature of the chemical substance/functional group in association with each other. Examples of the information regarding the features of the chemical substance/functional group include a CAS registry number, a molecular formula, a function and/or use (hereinafter, also referred to as "function/use") of the chemical substance/functional group, a method for modifying the chemical substance/functional group, a function/use of the chemical substance/functional group after modification, and information related to the function/use.

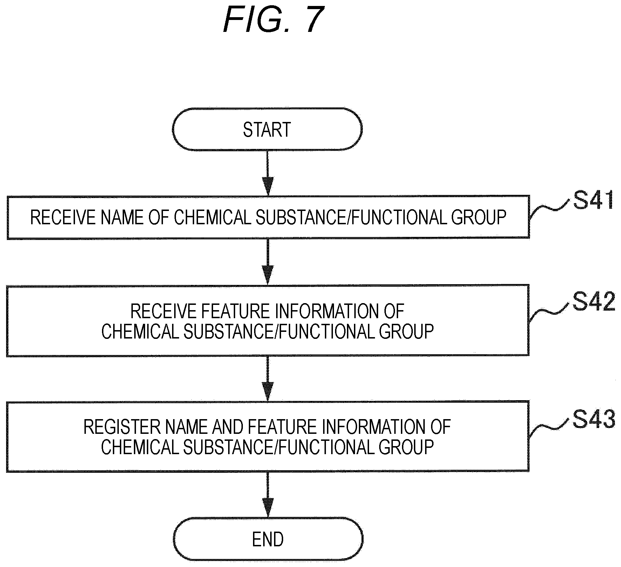

Here, an example of processing of registering data in the chemical substance/functional group database will be described with reference to Fig. 7. As illustrated in Fig. 7, the server 10 receives the name of the chemical substance/functional group (step S41), and then receives information regarding the features of the chemical substance/functional group (hereinafter, also referred to as "feature information of chemical substance/functional group") (step S42). The server 10 registers the name of the chemical substance/functional group received in step S41 and the feature information of the chemical substance/functional group received in step S42 in the chemical substance/functional group database in association with each other (step S43). These treatments may build and update a chemical substance/functional group database.

The chemical substance/functional group database may be stored within the server 10 (for example, the storage 13) or may reside external to the server 10.

An example of the operation of the information processing system 1 will be continuously described with reference to Fig. 4 again. The server 10 outputs the product characteristic information specified in step S14 (step S15). The server 10 may output the product characteristic information from an output apparatus connected to the input/output I/F15, or may output the product characteristic information to the user terminal 20 connected via the network 30.

### 2-2. Second Embodiment

An information processing system according to a second embodiment of the present disclosure will be described. A system configuration and a hardware configuration of an information processing system according to the second embodiment are the same as those of the first embodiment described in the above 2-1-1 and the above 2-1-2. Therefore, the description in the above 2-1-1 and the above 2-1-2 also applies to the second embodiment.

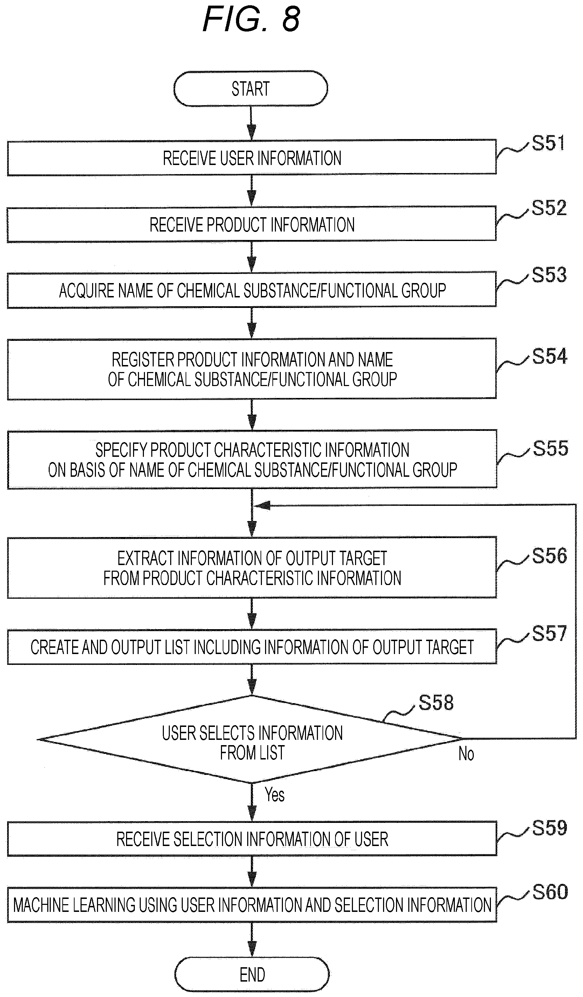

An example of an operation of the information processing system according to the second embodiment will be described with reference to Fig. 8. Differences from the first embodiment will be mainly described below. The points not described below may be the same as those of the first embodiment.

First, the server receives attribute information (hereinafter, also referred to as "user information") of the user (step S51). The processing in step S51 may be, for example, that the server receives the user information input by the user to the user terminal from the user terminal.

The user information may include, for example, a name (for example, a company name, an individual name, or a local government name), a location (for example, a company address or a home address), a business type, details of a business content, and the like.

Next, the server executes the processing of steps S52 to S55 illustrated in Fig. 8. Steps S52 to S55 are the same as steps S11 to S14 described in the first embodiment, and the description thereof also applies to steps S52 to S55.

Next, the server extracts information of the output target from the product characteristic information specified in step S55 (step S56). As an example of the process of step S56, the server may extract, as the information of the output target, information regarding a good using the function of the product and/or information regarding a good according to the use of the product from the product characteristic information. As a result, the server can output information regarding the good using the function of the product and/or information regarding the good according to the use of the product.

In an example of the process of above-described step S56, the server may extract the information of the output target on the basis of the characteristics of the good from the information regarding the good using the function of the product and/or the information regarding the good according to the use of the product. The characteristics of the good may include, for example, a function, a use, a transaction price of the good in a market, a main sales region, information regarding an environmental load caused by the good, and the like.

In an example of the process of above-described step S56, the server may extract the information of the output target on the basis of the characteristic of the good and the user information from the information regarding the good using the function of the product and/or the information regarding the good according to the use of the product. As a result, information more suitable for the user can be provided. For example, it is assumed that the information regarding the good using the function of the product includes information regarding a repellent capable of repelling the organism A living in a warm region and information regarding a repellent capable of repelling the organism B living in a cold region. That is, it is assumed that the product can be utilized as a new resource of a repellent for the organism A or a repellent for the organism B by modification. In this case, in a case where it is determined that the user lives in a warm region on the basis of the user information, the server may extract only the information regarding the repellent for the organism A suitable for the warm region as the information of the output target.

Next, the server creates a list including the information of the output target extracted in step S56, and outputs the list (step S57). For example, the server outputs the list to the user terminal.

In the present embodiment, the creation of the list may be selectively performed. For example, by creating a list in a case where there are a large number of pieces of information of the output target, it is possible to improve the visibility and convenience of the output information. In this case, the server may create the list on the basis of the user information or the output information regarding the good. For example, the server may display a good with a higher transaction price in the market, a good that can contribute more to environmental load reduction, or a good more suitable for the user in a higher order of the list.

Next, the user selects any one or more pieces of information from the list output in step S57 (step S58). For example, the user may select one or more pieces of information of interest from the product characteristic information included in the list. Note that, even in a case where the list is not created in above-described step S57 (for example, in a case where the number of pieces of information of the output target is small), the user may similarly select arbitrary one or more pieces of information from the output product characteristic information. In a case where the user does not select any information due to a reason that there is no information of interest or the like (step S58: No), the server executes the processing of steps S56 and S57 again. The server repeats these processes until the user selects one or more pieces of information.

In a case where the user selects one or more pieces of product characteristic information (step S58: Yes), the server receives one or more pieces of product characteristic information selected by the user from the list (product characteristic information) (step S59). Thereafter, the server performs machine learning using teacher data in which the user information and one or more pieces of product characteristic information selected by the user are associated with each other (step S60). As a result, in a case where the user information is input, it is possible to generate the learned model that extracts the information of the output target from the product characteristic information.

Examples of the type of the above-described machine learning include, but are not limited to, a support vector machine (SVM), a hidden Markov model (HMM), and a recurrent neural network (RNN).

In the present embodiment, the server may execute processing using the above-described learned model. Therefore, in the present embodiment, the server outputting the product characteristic information may be outputting the product characteristic information obtained by inputting the user information to the learned model. Specifically, the server may input the user information (the user information received in step S56) to the learned model in step S51, and output the obtained product characteristic information in subsequent step S57. In this way, by executing the processing using the learned model, information more suitable for the user or information more useful for the user can be output.

### 2-3. Modifications

An information processing system according to a modification of the present disclosure will be described. A system configuration and a hardware configuration of an information processing system according to the modification are the same as the configurations of the first embodiment described in the above 2-1-1 and the above 2-1-2. Therefore, the descriptions in the above 2-1-1 and the above 2-1-2 also apply to the present modification.

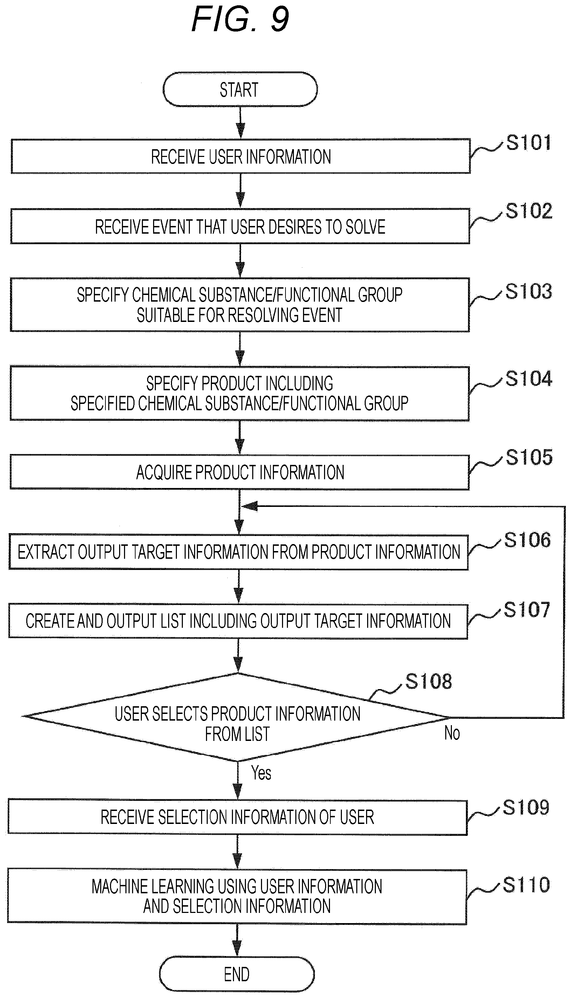

An example of an operation of the information processing system according to the modification will be described with reference to Fig. 9. Hereinafter, differences from the first embodiment or the second embodiment will be mainly described. The points not described below may be the same as those of the first embodiment or the second embodiment.

First, the server executes the processing of step S101 illustrated in Fig. 9. Step S101 is the same as step S51 described in the second embodiment, and the description of step S51 also applies to step S101.

Next, the server receives information regarding an event that the user desires to solve (step S102). The processing in step S102 may be, for example, that the server receives, from the user terminal, information regarding an event input by the user to the user terminal. Specific examples of the event that the user desires to solve include infection expansion of Chagas disease mediated by the Triatoma rubrofasciata, and feeding damage by the brown planthopper.

Next, the server identifies a chemical substance/functional group suitable for resolving the event (step S103). In step S103, the server may refer to, for example, a chemical substance/functional group database. The chemical substance/functional group database may be the same as that described in the first embodiment.

For example, it is assumed that acetophenone is registered in the chemical substance/functional group database, and repellency of Triatoma rubrofasciata and attraction of brown planthopper are registered as functions of acetophenone. For example, in a case where the event that the user desires to solve is the spread of Chagas disease mediated by Triatoma rubrofasciata, it is considered that acetophenone having a function of repelling Triatoma rubrofasciata is suitable for solving the event. In a case where the event that the user desires to solve is feeding damage by brown planthopper, acetophenone having a function of attracting brown planthopper is considered to be suitable for solving the event. In such a case, the server may refer to the chemical substance/functional group database in step S103 and identify acetophenone as a chemical substance suitable for resolving the event.

Next, the server identifies a product containing the identified chemical substance/functional group (step S104), and then acquires information related to the product (product information) (step S105). In steps S104 and S105, the server may refer to, for example, a product information database. The product information database may be the same as that described in the first embodiment.

For example, in a case where acetophenone is identified as the chemical substance suitable for solving the event in step S103, the server may refer to the product information database in steps S104 and S105, identify that the product containing acetophenone is a waste of, for example, an oyster-cultivation float constituted by polystyrene, and acquire information related to the waste (for example, information regarding a discharger that can provide the waste or the like).

Next, the server extracts information of the output target from the information acquired in step S105 (step S106), then creates a list including the information of the output target, and outputs the list (step S107). The user selects any one or more pieces of information from the output list (step S108). In a case where the user does not select any information (step S108: No), the server executes the processing of steps S106 and S107 again. The server repeats these processes until the user selects one or more pieces of information. In a case where the user selects one or more pieces of information (step S108: Yes), the server receives the one or more pieces of information selected by the user (step S109). Thereafter, the server performs machine learning using teacher data in which the user information and one or more pieces of information selected by the user are associated with each other (step S110). As a result, in a case where the user information is input, it is possible to generate the learned model that extracts the information of the output target from the product information.

Steps S106 to S110 are the same as steps S56 to S60 described in the second embodiment except that the type of information to be output and selected is different.

In the present modification, the server may execute processing using the above-described learned model. Therefore, in the present modification, the server outputting the product information may be outputting the product information obtained by inputting the user information to the learned model. Specifically, the server may input the user information (the user information received in step S101) to the learned model in step S106, and output the obtained product information in subsequent step S107. As described above, by executing the processing using the learned model, it is possible to select a chemical substance/functional group more suitable for solving the event presented by the user.

Furthermore, in the present modification, it is considered that machine learning of the correspondence relationship between the user information and the product information selected by the user can contribute to grasping what kind of event is desired to be resolved in a specific region.

The present disclosure can also have the following configurations.
[1] An information processing method executed by one or more processors, the method including:
   specifying at least one of information regarding a characteristic of a product described in following (a) to (c) on the basis of a name of a chemical substance and/or functional group contained in the product:
      (a) information regarding a characteristic different from an original characteristic of the product;
      (b) information regarding a characteristic that the product can acquire as it changes over time; and
      (c) information regarding a characteristic obtainable by modifying the product;
         and
   outputting the information regarding the characteristic of the product specified.
[2] The information processing method according to [1], further including
   receiving information regarding a product.
[3] The information processing method according to [2], in which the information regarding the product includes attribute information of the product and/or image information using the product as a subject.
[4] The information processing method according to [2] or [3], further including
   acquiring a name of a chemical substance and/or a functional group contained in the product received with reference to a database holding information regarding the product and the name of the chemical substance and/or the functional group contained in the product in association with each other.
[5] The information processing method according to [4], further including
   registering the information regarding the product received and the name of chemical substance and/or the functional group contained in the product received in the database in association with each other.
[6] The information processing method according to any one of [1] to [5], in which the information regarding the characteristic of the product includes information regarding a function and/or use of the product.
[7] The information processing method according to any one of [1] to [6], in which the outputting the information regarding the characteristic of the product specified includes outputting information regarding a good using a function of the product and/or information regarding a good according to a use of the product.
[8] The information processing method according to any one of [1] to [7], in which the outputting the information regarding the characteristic of the product specified includes extracting information of an output target on the basis of a characteristic of a good from among information regarding a good using a function of the product and/or information regarding a good according to a use of the product, creating a list including the information extracted of the output target, and outputting the list.
[9] The information processing method according to any one of [1] to [8], further including
   receiving attribute information of a user.
[10] The information processing method according to [9], in which the outputting the information regarding the characteristic of the product specified includes extracting information of an output target on the basis of a characteristic of a good and attribute information of the user from among information regarding a good using a function of the product and/or information regarding a good according to a use of the product, creating a list including the information extracted of the output target, and outputting the list.
[11] The information processing method according to [9] or [10], further including:
   receiving one or more pieces of information selected by the user from among the information regarding the characteristic of the product specified;
   performing machine learning using teacher data in which attribute information of the user and the one or more pieces of information selected by the user are associated with each other; and
   generating, in a case where attribute information of a user is input, a learned model for extracting information of an output target from among the information regarding the characteristic of the product specified.
[12] The information processing method according to [11], in which the outputting the information regarding the characteristic of the product specified includes outputting information obtained by inputting attribute information of the user to the learned model.
[13] The information processing method according to any one of [1] to [12], in which the product is an unnecessary product.
[14] The information processing method according to any one of [1] to [13], in which the product is a waste.
[15] An information processing system including:
   one or more processors; and one or more memories storing instructions that, when executed by the one or more processors, cause the information processing system to perform operations,
   in which the operations comprise:
      specifying at least one of information regarding a characteristic of a product described in following (a) to (c) on the basis of a name of a chemical substance and/or functional group contained in the product:
         (a) information regarding a characteristic different from an original characteristic of the product;
         (b) information regarding a characteristic that the product can acquire as it changes over time; and
         (c) information regarding a characteristic obtainable by modifying the product;
            and
      outputting the information regarding the characteristic of the product specified.

### REFERENCE SIGNS LIST

- 1: Information processing system
- 10: Server
- 20: User terminal
- 30: Network

## Claims

1. An information processing method executed by one or more processors, the method comprising:
specifying at least one of information regarding a characteristic of a product described in following (a) to (c) on a basis of a name of a chemical substance and/or functional group contained in the product:
(a) information regarding a characteristic different from an original characteristic of the product;
(b) information regarding a characteristic that the product can acquire as it changes over time; and
(c) information regarding a characteristic obtainable by modifying the product;
and
outputting the information regarding the characteristic of the product specified.

2. The information processing method according to claim 1, further comprising
receiving information regarding a product.

3. The information processing method according to claim 2, wherein the information regarding the product includes attribute information of the product and/or image information using the product as a subject.

4. The information processing method according to claim 2, further comprising
acquiring a name of a chemical substance and/or a functional group contained in the product received with reference to a database holding information regarding the product and the name of the chemical substance and/or the functional group contained in the product in association with each other.

5. The information processing method according to claim 4, further comprising
registering the information regarding the product received and the name of chemical substance and/or the functional group contained in the product received in the database in association with each other.

6. The information processing method according to claim 1, wherein the information regarding the characteristic of the product includes information regarding a function and/or use of the product.

7. The information processing method according to claim 1, wherein the outputting the information regarding the characteristic of the product specified includes outputting information regarding a good using a function of the product and/or information regarding a good according to a use of the product.

8. The information processing method according to claim 1, wherein the outputting the information regarding the characteristic of the product specified includes extracting information of an output target on a basis of a characteristic of a good from among information regarding a good using a function of the product and/or information regarding a good according to a use of the product, creating a list including the information extracted of the output target, and outputting the list.

9. The information processing method according to claim 1, further comprising
receiving attribute information of a user.

10. The information processing method according to claim 9, wherein the outputting the information regarding the characteristic of the product specified includes extracting information of an output target on a basis of a characteristic of a good and attribute information of the user from among information regarding a good using a function of the product and/or information regarding a good according to a use of the product, creating a list including the information extracted of the output target, and outputting the list.

11. The information processing method according to claim 9, further comprising:
receiving one or more pieces of information selected by the user from among the information regarding the characteristic of the product specified;
performing machine learning using teacher data in which attribute information of the user and the one or more pieces of information selected by the user are associated with each other; and
generating, in a case where attribute information of a user is input, a learned model for extracting information of an output target from among the information regarding the characteristic of the product specified.

12. The information processing method according to claim 11, wherein the outputting the information regarding the characteristic of the product specified includes outputting information obtained by inputting attribute information of the user to the learned model.

13. The information processing method according to claim 1, wherein the product is an unnecessary product.

14. The information processing method according to claim 1, wherein the product is a waste.

15. An information processing system comprising:
one or more processors; and one or more memories storing instructions that, when executed by the one or more processors, cause the information processing system to perform operations,
wherein the operations comprise:
specifying at least one of information regarding a characteristic of a product described in following (a) to (c) on a basis of a name of a chemical substance and/or functional group contained in the product:
(a) information regarding a characteristic different from an original characteristic of the product;
(b) information regarding a characteristic that the product can acquire as it changes over time; and
(c) information regarding a characteristic obtainable by modifying the product;
and
outputting the information regarding the characteristic of the product specified.
